(19) Europäisches Patentamt
European Patent Office
Office européen des brevets

(11) Veröffentlichungsnummer: **0 002 709**
**A1**

(12) # EUROPÄISCHE PATENTANMELDUNG

(21) Anmeldenummer: **78101628.2**

(22) Anmeldetag: **09.12.78**

(51) Int. Cl.²: **C 07 C 125/06,** C 07 C 155/02, C 07 C 155/08, A 01 N 9/20, A 01 N 9/12

(30) Priorität: **24.12.77 DE 2758021**

(43) Veröffentlichungstag der Anmeldung: **11.07.79 Patentblatt 79/14**

(84) Benannte Vertragsstaaten: **BE CH DE FR GB IT NL**

(71) Anmelder: **Bayer Aktiengesellschaft, Zentralbereich Patente,Marken und Lizenzen Bayerwerk, D-5090 Leverkusen 1 (DE)**

(72) Erfinder: **Knops, Hans-Joachim, Dr., Claudiusweg 3, D-5600 Wuppertal 1 (DE)**
Erfinder: **Brandes, Wilhelm, Dr., Eichendorffstrasse 3, D-5653 Leichlingen 1 (DE)**
Erfinder: **Scheinpflug, Hans, Dr., Am Thelenhof 15, D-5090 Leverkusen 1 (DE)**

(54) **Glykolsäure-Derivate, Verfahren zu ihrer Herstellung sowie ihre Verwendung als Fungizide.**

(57) Die Erfindung betrifft neue Glykolsäure-Derivate, ein Verfahren zu ihrer Herstellung sowie ihre Verwendung als Fungizide.

Die Verbindungen der Formel

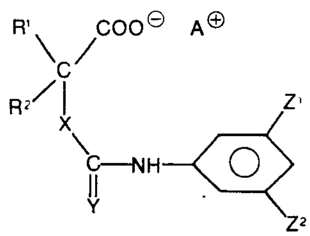

in welcher A, $R^1$, $R^2$, X, Y, $Z^1$ und $Z^2$ die in der Beschreibung angegebene Bedeutung besitzen, werden erhalten, wenn man Salze der Glykolsäure mit Phenylisocyanaten in Gegenwart eines Verdünnungsmittels umsetzt. Die erfindungsgemässen Wirkstoffe weisen eine starke fungitoxische Wirkung auf und können mit besonders gutem Erfolg zur Bekämpfung von Botrytis-Arten verwendet werden.

EP 0 002 709 A1

BAYER AKTIENGESELLSCHAFT
Zentralbereich
Patente, Marken und Lizenzen

5090 Leverkusen, Bayerwerk
Slr-by
Ia

Glykolsäure-Derivate, Verfahren zu ihrer Herstellung sowie ihre Verwendung als Fungizide

Die vorliegende Erfindung betrifft neue Glykolsäure-Derivate ein Verfahren zu ihrer Herstellung sowie ihre Verwendung als Fungizide.

Es ist bereits bekannt geworden, daß Thiuramdisulfide, wie beispielsweise Tetramethyl-thiuramdisulfid, gute fungizide Eigenschaften aufweisen (vergleiche US-Patentschrift 1 972 961 und Deutsches Reichspatent 642 532). Deren Wirksamkeit ist jedoch, insbesondere bei niedrigen Aufwandmengen und -konzentrationen, nicht immer ganz befriedigend.

Es wurden nun als neue Verbindungen die Glykolsäure-Derivate der Formel

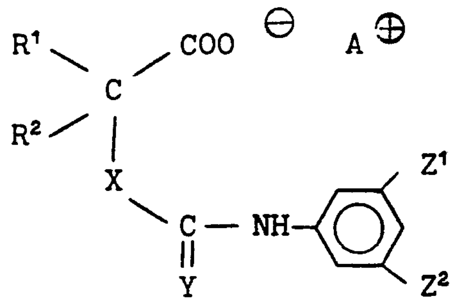

(I)

in welcher

A für ein Alkalimetall oder den Rest B - H steht, wobei B für ein tertiäres Amin steht,

$R^1$ und $R^2$ für Wasserstoff oder Alkyl stehen,

X und Y für Sauerstoff oder Schwefel stehen, und

$Z^1$ und $Z^2$ für Halogen stehen,

gefunden. Sie weisen starke fungizide Eigenschaften auf.

Weiterhin wurde gefunden, daß man die Glykolsäure-Derivate der Formel (I) erhält, wenn man Salze der Glykolsäure der Formel

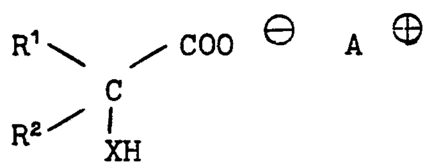

(II)

in welcher

A, $R^1$, $R^2$,
und X die oben angegebene Bedeutung haben,

mit Phenylisocyanaten der Formel

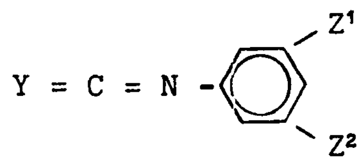

(III)

in welcher

Y, $Z^1$ und $Z^2$ die oben angegebene Bedeutung haben,

in Gegenwart eines Verdünnungsmittels umsetzt.

Ueberraschenderweise zeigen die erfindungsgemäßen Glykolsäure-Derivate eine erheblich höhere fungizide Wirkung, insbesondere gegen Botrytis-Arten, als das aus dem Stand der Technik bekannte Tetramethyl-thiuramdisulfid, welches ein anerkannt gutes Mittel gleicher Wirkungsrichtung ist. Die erfindungsgemäßen Wirkstoffe stellen somit eine Bereicherung der Technik dar.

Verwendet man das Triäthylammoniumsalz von Thioglykolsäure und 3,5-Dichlorphenylisocyanat als Ausgangsstoffe, so kann der Reaktionsablauf durch das folgende Formelschema wiedergegeben werden:

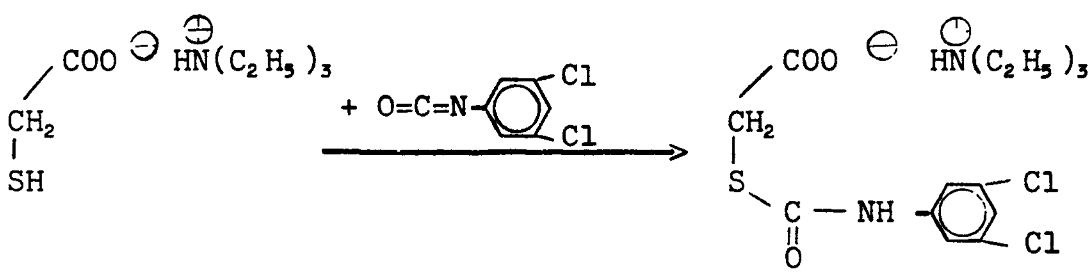

Die als Ausgangsstoffe zu verwendenden Glykolsäure-Derivate sind durch die Formel (II) allgemein definiert. In dieser Formel steht A vorzugsweise für ein Alkalimetall, wie beispielsweise Natrium oder Kalium sowie für den Rest B-H, wobei B vorzugsweise für ein tertiäres Amin, wie Pyridin oder

Trialkylamin mit 1 bis 4 Kohlenstoffatomen in jedem Alkylteil steht. $R^1$ und $R^2$ sind gleich oder verschieden und stehen vorzugsweise für Wasserstoff oder Alkyl mit 1 bis 4 Kohlenstoffatomen. X steht vorzugsweise für Sauerstoff oder Schwefel.

Die Ausgangsstoffe der Formel (II) sind allgemein bekannte Verbindungen der organischen Chemie. Man erhält sie in allgemein üblicher Weise, indem man die freien Glykolsäuren mit entsprechenden Basen versetzt. Die entstehenden Salze können ohne Isolierung direkt weiter umgesetzt werden (vergleiche auch Herstellungsbeispiele).

Die weiterhin für die erfindungsgemäße Umsetzung als Ausgangsstoffe benötigten Phenylisocyanate sind durch die Formel (III) allgemein definiert. In dieser Formel steht Y vorzugsweise für Sauerstoff oder Schwefel. $Z^1$ und $Z^2$ sind gleich oder verschieden und stehen vorzugsweise für Fluor, Chlor, Brom oder Jod.

Die Ausgangsstoffe der Formel (III) sind allgemein bekannte Verbindungen der organischen Chemie.

Für die erfindungsgemäße Umsetzung kommen als Lösungsmittel vorzugsweise inerte organische Lösungsmittel in Frage. Hierzu gehören vorzugsweise aromatische Kohlenwasserstoffe, wie Benzol, Toluol, Xylol oder 1,2-Dichlorbenzol und aliphatische, halogenierte Kohlenwasserstoffe, wie Methylenchlorid, Chloroform oder Tetrachlorkohlenstoff.

Die Reaktionstemperaturen können beim erfindungsgemäßen Verfahren in einem größeren Bereich variiert werden. Im allgemeinen arbeitet man zwischen 10 und 40°C, vorzugsweise bei Raumtemperatur.

Bei der Durchführung des erfindungsgemäßen Verfahrens arbeitet man vorzugsweise in molaren Mengen. Zur Isolierung der Verbindungen der Formel (I) wird das Lösungsmittel abdestilliert und der Rückstand nach üblichen Methoden aufgearbeitet.

Die erfindungsgemäßen Wirkstoffe weisen eine starke fungitoxische Wirkung auf. Sie schädigen Kulturpflanzen in den zur Bekämpfung von Pilzen notwendigen Konzentrationen nicht. Aus diesen Gründen sind sie für den Gebrauch als Pflanzenschutzmittel zur Bekämpfung von Pilzen geeignet. Fungitoxische Mittel im Planzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die erfindungsgemäßen Wirkstoffe haben ein breites Wirkungsspektrum und können angewandt werden gegen parasitäre Pilze, die oberirdische Pflanzenteile befallen oder die Pflanze vom Boden her angreifen, sowie gegen samenübertragbare Krankheitserreger. Eine besonders gute Wirksamkeit entfalten sie gegen parasitäre Pilze auf oberirdischen Pflanzenteilen.

Als Pflanzenschutzmittel können die erfindungsgemäßen Wirkstoffe mit besonders gutem Erfolg zur Bekämpfung von Botrytis-Arten, wie gegen den Erreger des Grauschimmels an Erdbeeren oder Trauben (Botrytis cinerea) verwendet werden.

Als Pflanzenschutzmittel können die erfindungsgemäßen Wirkstoffe zur Saatgut- oder Bodenbehandlung und zur Behandlung oberirdischer Pflanzenteile benutzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin—Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungiziden,Insektiziden,Akariziden,Nematiziden,Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertigen Lösungen, Emulsionen, Suspensionen, Pulver, Pasten und Granulate angewendet werden. Die Anwendung geschieht in üblicher Weise, z.B. durch Gießen, Spritzen, Sprühen, Stäuben, Streuen, Trockenbeizen, Feuchtbeizen, Naßbeizen, Schlämmbeizen oder Inkrustieren.

Bei der Verwendung als Blattfungizide können die Wirkstoffkonzentrationen in den Anwendungsformen in einem größeren Bereich variiert werden. Sie liegen im allgemeinen zwischen 0,1 und 0,000Cl Gewichtsprozent. Vorzugsweise zwischen 0,05 und 0,0001 %.

Bei der Saatgutbehandlung werden im allgemeinen Wirkstoffmengen von 0,001 bis 50 g je Kilogramm Saatgut, vorzugsweise 0,01 bis 10 g benötigt.

In entsprechenden Aufwandkonzentrationen zeigen die erfindungsgemäßen Wirkstoffe auch eine akarizide Wirkung.

Beispiel A

Botrytis-Test (Bohnen) / protektiv

Lösungsmittel: 4,7 Gewichtsteile Aceton
Dispergiermittel: 0,3 Gewichtsteile Alkyl-aryl-polyglykoläther
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man Pflanzen von Phaseolus vulgaris im 2-Blattstadium bis zur Tropfnässe. Nach 24 Stunden werden auf jedes Blatt 2 kleine mit Botrytis cinerea bewachsene Agarstücke aufgelegt. Die inokulierten Pflanzen werden in einer abgedunkelten, feuchten Kammer bei 20°C aufgestellt. 3 Tage nach der Inokulation wird die Größe der Befallsflecken auf den Blättern bonitiert.

Die erhaltenen Boniturwerte werden auf Prozent Befall umgerechnet. 0 % bedeutet keinen Befall, 100 % bedeutet, daß der Befallsfleck vollständig ausgebildet ist.

Wirkstoff, Wirkstoffkonzentrationen und Ergebnisse werden ermittelt.
In diesem Test zeigt z.B. folgende Verbindung eine sehr gute Wirkung, die derjenigen der aus dem Stand der Technik bekannten Verbindungen überlegen ist:
Verbindung gemäß Herstellungsbeispiel 3.

Herstellungsbeispiele

Beispiel 1

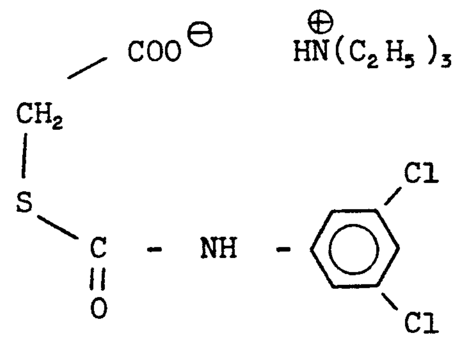

4,6g (0,05 Mol) Thioglykolsäure werden mit 5,05 g (0,05 Mol) Triäthylamin neutralisiert, wobei sich das Reaktionsgemisch schwach erwärmt. Das entstandene Salz wird in 100 ml Toluol gelöst und mit 9,4 g (0,05 Mol) 3,5-Dichlorphenylisocyanat in 200 ml Toluol versetzt. Man läßt zwei Stunden bei Raumtemperatur rühren und saugt anschließend den weißen, kristallinen Niederschlag ab. Man erhält 14g (74 % der Theorie) Triäthylammoniumsalz der 3,5-Dichlorphenylcarbamoylthioglykolsäure vom Schmelzpunkt 132-34°C.

Analog können die Beispiele der nachfolgenden Tabelle 1 erhalten werden.

Tabelle 1

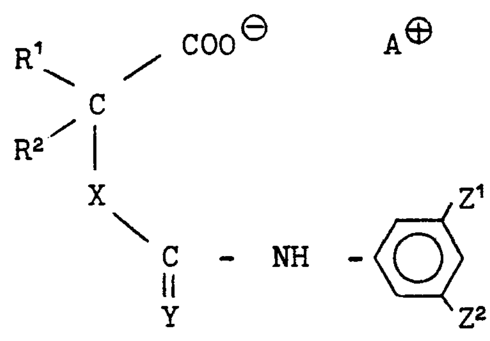

| Bsp. Nr. | $R^1$ | $R^2$ | X | Y | $Z^1$ | $Z^2$ | A | Schmelzpunkt (°C) |
|---|---|---|---|---|---|---|---|---|
| 2 | $CH_3$ | H | S | S | Cl | Cl | $HN(C_2H_5)_3$ | 215 |
| 3 | $CH_3$ | $CH_3$ | S | O | Cl | Cl | $HN(C_2H_5)_3$ | 230 |

In entsprechender Weise können noch die nachfolgend aufgeführten Verbindungen der allgemeinen Formel I erhalten werden:

| Beispiel Nr. | $R^1$ | $R^2$ | X | Y | $Z^1$ | $Z^2$ | A | Schmelzpunkt (°C) |
|---|---|---|---|---|---|---|---|---|
| 4 | H | H | S | S | Cl | Cl | $HN(C_2H_5)_3$ | |
| 5 | H | H | S | O | Cl | Cl | HN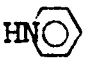 | 103-105 |
| 6 | H | H | S | S | Cl | Cl | HN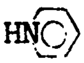 | |
| 7 | H | H | S | O | Cl | Cl | Na | 175-181 |
| 8 | H | H | S | S | Cl | Cl | Na | |
| 9 | $CH_3$ | H | S | O | Cl | Cl | $HN(C_2H_5)_3$ | 109-111 |
| 10 | $CH_3$ | H | S | S | Cl | Cl | HN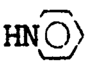 | |
| 11 | $CH_3$ | H | S | O | Cl | Cl | HN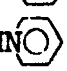 | |
| 12 | $CH_3$ | H | S | S | Cl | Cl | Na | |
| 13 | $CH_3$ | H | S | O | Cl | Cl | Na | |
| 14 | $CH_3$ | $CH_3$ | S | S | Cl | Cl | $HN(C_2H_5)_3$ | |
| 15 | $CH_3$ | $CH_3$ | S | O | Cl | Cl | HN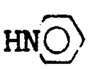 | |
| 16 | $CH_3$ | $CH_3$ | S | S | Cl | Cl | HN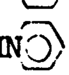 | |
| 17 | $CH_3$ | $CH_3$ | S | O | Cl | Cl | Na | |
| 18 | $CH_3$ | $CH_3$ | S | S | Cl | Cl | Na | |
| 19 | $CH_3$ | $CH_3$ | O | O | Cl | Cl | $HN(C_2H_5)_2$ | 146-149 |
| 20 | $CH_3$ | $CH_3$ | O | O | Cl | Cl | Na | 203-210 (Zers.) |
| 21 | H | H | O | O | Cl | Cl | $HN(C_2H_5)_2$ | 166-188 |

PATENTANSPRÜCHE:

1.) Glycolsäure-Derivate der Formel

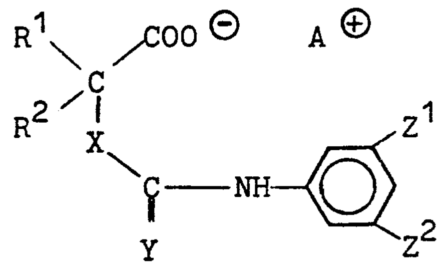

(I)

in welcher

A für ein Alkalimetall oder den Rest B - H steht, wobei B für ein tertiäres Amin steht,

$R^1$ und $R^2$ für Wasserstoff oder Alkyl stehen,

X und Y für Sauerstoff oder Schwefel stehen, und

$Z^1$ und $Z^2$ für Halogen stehen.

2.) Verfahren zur Herstellung von Glycolsäure-Derivaten, dadurch gekennzeichnet, daß man Salze der Glykolsäure der Formel

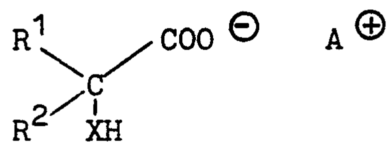

(II)

in welcher
A, $R^1$, $R^2$ und X die oben angegebene Bedeutung haben,

mit Phenylisocyanaten der Formel

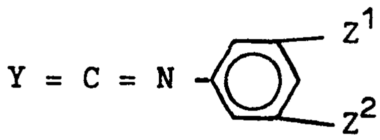

(III)

in welcher
Y, $Z^1$ und $Z^2$ die oben angegebene Bedeutung haben, in Gegenwart eines Verdünnungsmittels umsetzt.

3.) Fungizide Mittel, gekennzeichnet durch einen Gehalt an mindestens einem Glycolsäure-Derivat gemäß Anspruch 1.

4.) Verfahren zur Bekämpfung von Pilzen, dadurch gekennzeichnet, daß man Glycolsäure-Derivate gemäß Anspruch 1 auf Pilze oder ihren Lebensraum einwirken läßt.

5.) Verwendung von Glycolsäure-Derivaten gemäß Anspruch 1 zur Bekämpfung von Pilzen.

6.) Verfahren zur Herstellung von fungiziden Mitteln, dadurch gekennzeichnet, daß man Glycolsäure-Derivate gemäß Anspruch 1 mit Streckmitteln und/oder oberflächenaktiven Mitteln vermischt.

Europäisches Patentamt

**EUROPÄISCHER RECHERCHENBERICHT**

0002709

Nummer der Anmeldung

EP 78 10 1628

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| X | DE - A - 2 021 327 (SUMITOMO)<br>* Ansprüche 1,5,6 *<br>-- | 1-6 | C 07 C 125/06<br>155/02<br>155/08<br>A 01 N 9/20<br>9/12 |
| X | US - A - 3 876 796 (FUJINAMI et al)<br>* Zusammenfassung *<br>---- | 1-6 | |
| | | | RECHERCHIERTE SACHGEBIETE (Int. Cl.²) |
| | | | C 07 C 125/06<br>155/02<br>155/08 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| ⁄ | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Den Haag | 27-03-1979 | GAUTIER |